(19) Europäisches Patentamt
European Patent Office
Office européen des brevets

(11) EP 1 471 067 A1

(12) DEMANDE DE BREVET EUROPEEN

(43) Date de publication:
**27.10.2004 Bulletin 2004/44**

(51) Int Cl.[7]: **C07D 513/04**, C07D 513/14, A61K 31/4985, A61K 31/5365

(21) Numéro de dépôt: **04290957.2**

(22) Date de dépôt: **09.04.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **25.04.2003 FR 0305118**

(71) Demandeur: **L'OREAL**
**75008 Paris (FR)**

(72) Inventeurs:
- **Erdelmeier, Irène**
  **75013 Paris (FR)**
- **Lucet-Levannier, Karine**
  **92500 Reuil-Malmaison (FR)**

(74) Mandataire: **Renard, Emmanuelle**
**L'OREAL - D.I.P.I.**
**25-29 Quai Aulagnier**
**92600 Asnières (FR)**

# (54) Dérivés hétérocycliques de l'acide 2-oxothiazolidine 4-carboxylique, utilisation comme agent de photoprotection active

(57) La présente invention se rapporte à de nouveaux dérivés hétérocycliques de l'acide 2-oxothiazolidine 4-carboxylique de formule (I) suivante

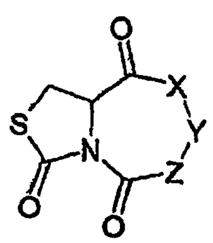

(I)

à leur procédé de synthèse et aux compositions les contenant.

L'invention a encore pour objet l'utilisation d'au moins un dérivé hétérocyclique de l'acide 2-oxothiazolidine 4-carboxylique dans une composition ou pour la fabrication d'une composition, le dit dérivé ou ladite composition étant destiné à induire une photo-protection active de la peau, avantageusement à préparer la peau à recevoir le soleil et/ou à protéger la peau et ses annexes durant ou suite à une exposition aux UV.

Un autre objet de l'invention se rapporte encore à un procédé de traitement cosmétique de la peau par administration orale ou application topique cutanée d'une composition cosmétique telle que définie précédemment.

EP 1 471 067 A1

## Description

La présente invention se rapporte à de nouveaux dérivés hétérocycliques de l'acide 2-oxothiazolidine 4-carboxylique, à leur procédé de synthèse et aux compositions les contenant.

L'invention a encore pour objet l'utilisation d'au moins un dérivé hétérocyclique de l'acide 2-oxothiazolidine 4-carboxylique dans une composition ou pour la fabrication d'une composition le dit dérivé ou ladite composition étant destiné à induire une photo-protection active de la peau, avantageusement à préparer la peau à recevoir le soleil et/ou à protéger la peau et ses annexes durant ou suite à une exposition aux UV.

Un autre objet de l'invention se rapporte encore à un procédé de traitement cosmétique de la peau par administration orale ou application topique cutanée d'une composition cosmétique telle que définie précédemment.

Afin de protéger la peau contre les effets néfastes du rayonnement solaire, il est bien connu d'apporter une photoprotection artificielle par l'application topique cutanée d'agents empêchant l'action des UV de deux manières :

- par absorption de l'énergie apportée par les photons; ce sont les filtres solaires classiques organique et/ou inorganique actif dans l'UVA et/ou l'UVB;
- par diffraction de la lumière par un écran physique tel que le dioxyde de titane par exemple. Les crèmes « écran total » contiennent en général un tel produit plus un filtre chimique.

On peut également apporter des agents antioxydants tels que la vitamine E, les caroténoïdes ou le glutathion, qui peuvent avoir un intérêt en vue de réduire les effets du stress oxydatif au niveau cellulaire. Ces agents induisent une photo-protection active en stimulant les systèmes de défense naturelle des cellules de la peau vis-à-vis des dommages induits par les irradiations UV.

L'utilisation de l'acide 2-oxothiazolidine 4-carboxylique a fait déjà l'objet de nombreuses études et brevets, notamment dans le but de protéger l'organisme contre différents stress. Ainsi, Il est connu du document US 5,208,249 (Clintec) que l'acide 2-oxothiazolidine 4-carboxylique et ses esters sont capables de stimuler la synthèse de glutathion dans certaines cellules du corps humain. Le glutathion qui se retrouve dans toutes les cellules des eucaryotes est un tripéptide composé des trois acides aminés suivants : acide glutamique, cystéine et glycine. Le glutathion est notamment connu pour intervenir dans la protection des cellules contre différents dommages tels que ceux dus aux radicaux libres (US 5,208,249) et pour jouer un rôle central dans les systèmes de défenses antioxydant contre les radicaux libres et les composés toxiques d'origine endogène et exogène (J. Med. Chem., 1999, Vol. 42, N°23, pp. 4733-4740). En outre, il est connu du document EP 0 655 245 (Free Radical Science) d'utiliser une composition contenant l'acide 2-oxothiazolidine 4-carboxylique ou ses esters pour retarder le processus de vieillissement chez les mammifères, en maintenant des niveaux intracellulaires en glutathion en une quantité suffisante pour prévenir les dommages oxydatifs causés par les radicaux libres au niveau des cellules.

Il n'en demeure pas moins que le désir de protéger la peau et ses annexes contre le stress oxydatif et photo-oxydatif afin de maintenir ou d'améliorer l'apparence cutanée d'un individu conduit toujours à la recherche incessante de nouveaux agents efficaces pour protéger la peau et ses annexes, les dits agents devant par ailleurs être bien métabolisés et formulables dans des compositions, notamment cosmétiques.

Le procédé de synthèse d'un dérivé hétérocyclique de formule (II) a bien été rapporté dans l'art antérieur (Bull. Chem. Soc. Jpn., 1964, 37 (2), pp. 242-244), mais ce dérivé n'est pas décrit comme un agent stimulant la synthèse intracellulaire de glutathion.

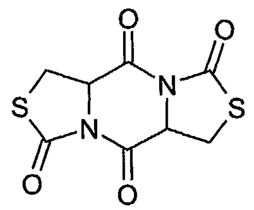

(II)

La demanderesse a maintenant découvert, de manière surprenante et inattendue de nouveaux dérivés hétérocycliques de l'acide 2-oxothiazolidine 4-carboxylique qui sont des agents susceptibles d'induire une photo-protection active de la peau, grâce à la stimulation de la synthèse intracellulaire de glutathion, ce qui permet de préparer la peau à recevoir le soleil et/ou de protéger la peau et ses annexes durant ou suite à une exposition aux UV.

La présente invention est basée sur l'observation de la demanderesse selon laquelle les nouveaux dérivés hétérocycliques de l'acide 2-oxothiazolidine 4-carboxylique de formule (I) tels que définis ci-dessous constituent d'excellents agents stimulant la synthèse intracellulaire de glutathion.

L'invention a donc pour objet de nouveaux dérivés de l'acide 2-oxothiazolidine 4-carboxylique répondant à la formule (I) suivante :

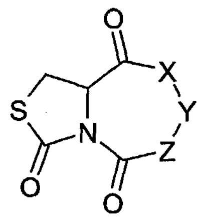

(I)

dans laquelle,

- X représente un atome d'oxygène ou un radical NR";
    - R" représente un radical alkyle ayant de 1 à 12 atomes de carbone, avantageusement de 1 à 6 atomes de carbone, linéaire, cyclique ou ramifié, éventuellement substitué; de préférence par au moins un radical -COOH; $-NH_2$; -OH, et/ou -SH;
- Y représente un groupement $(CH_2)_n$ ;
- Z représente un groupement (CRR')
    - n est un nombre entier choisi parmi 0, 1, 2 ou 3, de préférence 0 ou 1, avantageusement n est égal à 0;
    - R et R', identiques ou différents représentent :
        - un atome d'hydrogène;
        - un radical $-Si-(CH_3)_3$ ; $-Si(CH_3)_2-OH$ ; $-Si-(CH_3)_2-OEt$, $-Si-(CH_3)_2-O-CH_2-Ph$ ; $-Si-(iPr)_2-OEt$ ;
        - un halogène;
        - un radical alkyle tel que défini précédemment,
        - un radical aryle, éventuellement substitué;
        - un radical arylalkyle, éventuellement substitué;

    étant entendu que lorsque X représente un radical NR", R" et R pris ensemble peuvent former avec les 2 atomes les portant, un hétérocycle ayant 4, 5 ou 6 atomes de carbone, de préférence 4 atomes de carbone;
- ou Y et Z représentent chacun un atome de carbone et forment ensemble un cycle un hétérocycle aromatique ayant 5, 6 ou 7 atomes de carbone, de préférence 6 atomes de carbone, le dit cycle ou hétérocycle étant éventuellement substitué;

ses isomères optiques et/ou géométriques, seuls ou en mélange en toutes proportions ainsi que ses sels physiologiquement acceptables.

L'invention concerne donc également les isomères optiques et/ou géométriques des dérivés de formule (I), seuls ou en mélange en toutes proportions, ainsi que les sels physiologiquement acceptables de ces dérivés.

Comme sels du dérivé de formule (I), on peut citer les sels obtenus par addition du dérivé de formule (I) avec un acide inorganique, choisi notamment parmi, les acides chlorhydrique, sulfurique, nitrique, carbonique, et phosphorique, ou avec un acide organique, choisi en particulier parmi les acides succinique, fumarique, lactique, glycolique, citrique, gluconique, salicylique et tartrique.

Au sens de l'invention, le terme radical aryle s'entend comme un cycle aromatique ayant 5 ou 6 chaînons ou un hétérocycle aromatique ayant 5 ou 6 chaînons. Un radical aryle particulièrement préféré selon l'invention est le radical phényle.

Par radical arylalkyle, on entend de préférence selon l'invention les radicaux alkyles-aryles ayant de 6 à 12 atomes de carbone, définition dans laquelle le terme aryle s'entend comme un cycle aromatique ayant 5 ou 6 chaînons ou un hétérocycle aromatique ayant 5 ou 6 chaînons. Préférentiellement selon l'invention, le radical arylalkyle est en $C_7-C_{10}$. Un radical arylalkyle particulièrement préféré selon l'invention est le radical benzyle.

Par radical aryle substitué ou radical arylalkyle substitué, on entend un radical, dont la partie aromatique est substitué au moins un groupement choisi parmi un groupement hydroxy (-OH), un groupement cyano (-CN), un grou-

pement trifluorométhyle (-$CF_3$), un radical méthoxy (-$OCH_3$) ou un atome d'halogène. L'atome d'halogène peut être choisi parmi le chlore, le brome, le fluor, l'iode.

Un radical aryle substitué préféré selon l'invention est le radical phényle substitué par groupement un radical alkyle, un groupement hydroxy ou un radical méthoxy.

Un radical arylalkyle substitué préféré selon l'invention est un radical benzyle substituée par au moins un groupement choisi parmi un radical alkyle, un groupement hydroxy ou un radical méthoxy.

Préférentiellement selon l'invention, le radical alkyle est en $C_1$-$C_4$ et est choisi parmi les radicaux méthyle, éthyle, propyle, isopropyle, butyle, isobutyle ou tertiobutyle et plus particulièrement les radicaux méthyle ou éthyle.

Selon une forme d'exécution préférée de l'invention, le dérivé de formule (I) est tel que l'une au moins des conditions suivantes, et de préférence toutes ces conditions, sont satisfaites :

- X représente un atome d'oxygène ou un radical NR'';
- n est égal à 0;
- Z représente un groupement (CRR')
- R et R'', identiques ou différents, représentent un atome d'hydrogène, un radical alkyle tel que défini précédemment, ou un cycloakyle;
- R' représente un atome d'hydrogène, un radical phényle, un radical benzyle ou la chaîne latérale d'un acide aminé, de préférence choisie parmi celle de l'alanine, l'isoleucine, la leucine, la méthionine, le phénylalanine, le tryptophane, la valine, l'asparagine, la cystéine, la glutamine, le glycocolle, la sérine, le thréonine, l'acide aspartique, l'acide glutamique, l'arginine, l'histidine, la lysine;

étant entendu que R'' et R pris ensemble peuvent former avec les 2 atomes les portant, la chaîne latérale de la proline, ou,

- Y et Z représentent chacun un atome de carbone et forment ensemble un cycle aromatique éventuellement substitué, ayant 6 atomes de carbone.

Les composés préférés suivant l'invention sont choisis parmi les composés suivants:

- Tetrahydro-thiazolo[3,4-a]pyrazine-3,5,8-trione ;
- 6-Methyltetrahydro[1,3]thiazolo[3,4a]pyrazine-3,5,8trione ;
- 6-Ethyltetrahydro[1,3]thiazolo[3,4a]pyrazine-3,5,8trione ;
- 6-Propyltetrahydro[1,3]thiazolo[3,4a]pyrazine-3,5,8trione ;
- 6-*iso*-Propyltetrahydro[1,3]thiazolo[3,4a]pyrazine-3,5,8trione ;
- 6-Propyltetrahydro[1,3]thiazolo[3,4a]pyrazine-3,5,8-trione ;
- 6-[1-Methylpropyl]tetrahydro[1,3]thiazolo[3,4-a]pyrazine-3,5,8-trione ;
- 6-isobutyltétrahydro[1,3]thiazolo[3,4a]pyrazine-3,5,8-trione ;
- 6-Phenyltetrahydro[1,3]thiazolo[3,4a]pyrazine-3,5,8-trione ;
- 6-Benzyltetrahydro[1,3]thiazolo[3,4a]pyrazine-3,5,8-trione ;
- 6-(Methylsulfanyl)ethyl)tetrahydro[1,3]thiazolo[3,4a]pyrazine-3,5,8-trione ;
- 6-(Ethylsulfanyl)methyl)tetrahydro[1,3]thiazolo[3,4a]pyrazine-3,5,8-trione ;
- 7-Methyltetrahydrotetra[1,3]thiazolo[3,4-a]pyrazine-3,5,8-trione ;
- 7-Ethyltetrahydrotetra[1,3]thiazolo[3,4-a]pyrazine-3,5,8-trione ;
- Tetrahydro-1H,5H-pyrrolo[1,2a][1,3]thiazolo[3,4d]pyrazine,3,5,10(10aH)-trione ;
- 3-(3,5,8-Trioxo-hexahydro-thiazolo[3,4-a]pyrazin-6-yl)-propionic acid ;
- 6-Mercaptomethyl-tetrahydro-thiazolo[3,4-a]pyrazine-3,5,8-trione ;
- 7-Methyltétrahydro-thiazolo[3,4-a]pyrazine-3,5,8-trione ;
- 6,7-Dimethyltetrahydro[1,3]thiazolo[3,4a]pyrazine-3,5,8trione ;
- 6-Ethyl,7-methyltetrahydro[1,3]thiazolo[3,4a]pyrazine-3,5,8trione ;
- 6-Propyl,7-methyltetrahydro[1,3]thiazolo[3,4a]pyrazine-3,5,8trione ;
- 6-*iso*-Propyl,7-methyltetrahydro[1,3]thiazolo[3,4a]pyrazine-3,5,8trione ;
- 6-Propyl,7-methyltetrahydro[1,3]thiazolo[3,4a]pyrazine-3,5,8-trione ;
- 6-[1-Methylpropyl],7-methyltetrahydro[1,3]thiazolo[3,4-a]pyrazine-3,5,8-trione ;
- 6-*iso*-Butyl,7-methyltétrahydro[1,3]thiazolo[3,4a]pyrazine-3,5,8-trione ;
- 6-Phenyl,7-methyltetrahydro[1,3]thiazolo[3,4a]pyrazine-3,5,8-trione ;
- 6-Benzyl,7-methyltetrahydro[1,3]thiazolo[3,4a]pyrazine-3,5,8-trione ;
- 6-(Methylsulfanyl)ethyl),7-methyltetrahydro[1,3]thiazolo[3,4a]pyrazine-3,5,8-trione;
- 6-(Ethylsulfanyl)methyl),7-methytetrahydro[1,3]thiazolo[3,4a]pyrazine-3,5,8-trione;

- 3-(3,5,8-Trioxo-hexahydro-7-methyl-thiazolo[3,4-a]pyrazin-6-yl)-propionic acid ;
- 6-Mercaptomethyl-7-methyl-tetrahydro-thiazolo[3,4-a]pyrazine-3,5,8-trione ;
- 1H-[1,3]thiazolo[4,3-c]oxazine-3,5,8[6H,8aH]-trione ;
- 1,10a-Dihydro-9-oxa-2-thia-3a-aza-benzo[f]azulène-3,4,10-trione ;
- 6-Octanoyl-1,10a-dihydro-9-oxa-2-thia-3a-aza-benzo[f]azulène-3,4,10-trione ;
- (6-Carboxymethyl-3,5,8-trioxo-tetrahydro-thiazolo[4,3-c][1,4]oxazin-6-yl)-acetic acid,

leurs isomères optiques et/ou géométriques, seuls ou en mélange en toutes proportions, ainsi que leurs sels physiologiquement acceptables.

Les composés particulièrement préférés suivant l'invention sont choisis parmi les composés suivants:

- 1 H-[1,3]thiazolo[4,3-c]oxazine-3,5,8[6H,8aH]-trione ;
- Hexahydro-pyrolo[1,2-a]thiazolo[3,4-d]pyrazine-3,5,10-trione ;
- 6-Benzyl-tétrahydro-thiazolo[3,4-a]pyrazine-3,5,8-trione ;
- Tétrahydro-thiazolo[3,4-a]pyrazine-3,5,8-trione ;
- 6-Phenyl-tétrahydro-thiazolo[3,4-a]pyrazine-3,5,8-trione;

leurs isomères optiques et/ou géométriques, seuls ou en mélange en toutes proportions, ainsi que leurs sels physiologiquement acceptables.

Ainsi, un autre objet de l'invention se rapporte à l'utilisation d'au moins un dérivé hétérocyclique de l'acide 2-oxothiazolidine 4-carboxylique tel que défini précédemment dans une composition ou pour la fabrication d'une composition, le dit dérivé ou ladite composition étant destiné à induire une photo-protection active de la peau, avantageusement à préparer la peau à recevoir le soleil et/ou à protéger la peau et ses annexes durant ou suite à une exposition aux UV.

Par annexe de la peau on entend par exemple les cheveux et les ongles.

Un autre objet de l'invention se rapporte encore à l'utilisation cosmétique d'au moins un composé de formule (I) tel que défini ci-dessus, comme agent stimulant la synthèse intracellulaire de glutathion et/ou maintenant un niveau intracellulaire en glutathion en une quantité suffisante pour prévenir les dommages oxydatifs causés par les radicaux libres au niveau des cellules.

Le vieillissement cutané résulte de deux processus distincts et indépendants qui font intervenir des facteurs intrinsèques ou extrinsèques. Le vieillissement intrinsèque ou chronobiologique correspond au vieillissement « normal » ou physiologique lié à l'age. Le vieillissement extrinsèque correspond au vieillissement provoqué d'une manière générale par l'environnement et plus particulièrement au photovieillissement dû à l'exposition au soleil, à la lumière ou à tout autre rayonnement (EP-A2-0 815 840, Kligman, A. M. *et al.,* Journal of Cutaneous Aging and Cosmetic Dermatology, Vol. 1, N°.1, pp. 5-12 (1988)).

Le vieillissement cutané qui résultent des effets sur la peau de facteurs intrinsèques ou extrinsèques, se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté et par l'apparition de télangiectasies.

Grâce à la stimulation de la synthèse intracellulaire de glutathion et/ou en maintenant un niveau intracellulaire en glutathion les dérivés de formule (I) suivant l'invention permettent de prévenir ou de réduire les effets sur la peau des facteurs intrinsèques ou extrinsèques précédemment décrits.

Avantageusement, l'invention se rapporte donc à l'utilisation d'au moins un dérivé de formule (1) tel que défini précédemment dans une composition ou pour la fabrication d'une composition, le dit dérivé ou ladite composition étant destiné à lutter contre le vieillissement cutané intrinsèque et/ou extrinsèque.

En outre, les dérivés de formule (I), avantageusement les dérivés pour lesquels X représente un atome d'oxygène tels que définis précédemment, constituent d'excellents actifs pour favoriser la desquamation de la peau et/ou stimuler le renouvellement épidermique et permettent de lutter encore plus efficacement contre le vieillissement intrinsèque et/ou intrinsèque de la peau.

Un autre objet de l'invention se rapporte à l'Utilisation d'au moins un dérivé de formule (I) tel que défini précédemment dans une composition ou pour la fabrication d'une composition, le dit dérivé ou ladite composition étant destiné à favoriser la desquamation de la peau et/ou stimuler le renouvellement épidermique et est avantageusement destinée à lutter contre le vieillissement cutané intrinsèque et/ou extrinsèque.

Le procédé de fabrication des dérivés de formule (I) suivant l'invention suit le schéma général de synthèse qui est présentée sur la figure 1 (Fig. 1).

Les produits de départ sont l'acide 2-oxothiazolidine 4-carboxylique et un acide aminé, un hydroxyacide ou un halogénoacide dont la fonction acide est protégée (P*).

L'acide aminé de départ est un acide alpha, béta ou gamma aminé.

L'hydroxyacide de départ est un acide alpha, béta ou gamma hydroxylé.
L'halogénoacide de départ est un acide alpha, béta ou gamma halogéné.

L'acide aminé, l'hydroxyacide ou l'halogénoacide de départ sont obtenus sous forme protégée suivant des méthodes classiquement utilisées en synthèse organique bien connues de l'homme du métier, par exemple celles décrites dans le document *Protective Groups in Organic Synthesis,* T. W. Greene, P. G. M. Wuts (Wiley Interscience). L'acide aminé, l'hydroxyacide ou l'halogénoacide libre ou sous forme protégée peuvent également être obtenu directement auprès de fournisseurs.

Les dérivés de formule (I) suivant l'invention sont obtenus suivant des méthodes de couplage peptidique classiquement utilisées et bien connues de l'homme du métier comme par exemple celles décrites dans le document *Peptide Chemistry - A Practical Textbook,* Miklos Bodanszky, 1988 (Springler Verlag).

Ainsi, un autre objet de l'invention se rapporte encore à un procédé de fabrication des dérivés de formule (I) telle que définie précédemment, à partir de l'acide L-2-oxothiazolidine 4-carboxylique, caractérisé en ce qu'il comprend les étapes suivantes:

i) Réaction de couplage, en présence d'une base entre l'acide 2-oxothiazolidine 4-carboxylique et un acide aminé, un hydroxyacide ou un halogénoacide dont la fonction acide est protégée;
ii) Réaction de déprotection par un agent acide de la fonction acide protégée du produit obtenu à l'issu de l'étape i);
iii) Réaction de cyclisation intramoléculaire de type peptidique;

étant entendu que l'étape iii) est mise en oeuvre si la cyclisation intramoléculaire ne se produit spontanément à l'étape ii).

Selon une forme d'exécution préférée de l'invention, le procédé est tel que l'une au moins des conditions suivantes, et de préférence toutes ces conditions, sont satisfaites:

Lorsque le produit de départ est un halogénoacide l'étape i) consiste à:

- placer l'acide 2-oxothiazolidine 4-carboxylique dans un solvant polaire, de préférence anhydre, de préférence l'acétonitrile ou la DMF;
- ajouter au moins une base organique ou inorganique, de préférence le carbonate de potassium, de préférence 0.1 à 2 équivalents;
- ajouter un halogénoacide, de préférence un bromoacide, dont la fonction acide est protégée, de préférence de 1 à 3 équivalents.

Lorsque le produit de départ est un acide aminé ou un hydroxyacide l'étape i) consiste à:

- placer l'acide 2-oxothiazolidine 4-carboxylique dans un solvant polaire, de préférence anhydre, de préférence l'acétonitrile;
- ajouter au moins un agent de couplage, de préférence 1 équivalent et/ou un additif pour supprimer l'énantiomérisation lors du couplage, de préférence 1 équivalent.
- ajouter un acide aminé ou un hydroxyacide dont la fonction acide est protégée, de préférence 1 équivalent, et une base, de préférence une amine tertiaire, avantageusement la triéthylamine, de préférence 1 équivalent. Lorsque l'acide aminé dont la fonction acide est protégée, est sous forme de chlorhydrate on utilise 2 équivalents de base, de préférence de triéthylamine.

On utilise un additif pour supprimer l'énantiomérisation lors du couplage lorsque l'acide aminé de ou l'hydroxyacide de départ sont optiquement purs.

L'étape ii) consiste à:

- placer le produit obtenu à l'issu de l'étape i), de préférence 1 équivalent, dans un solvant aprotique;
- ajouter un agent acide, avantageusement de l'acide trifluoroacétique, de préférence 5 équivalents, avantageusement en présence d'un silane, de préférence le triéthylsilane ou le triisopropylsilane, de préférence 1 équivalent.

Les produits obtenus à l'issu de l'étape ii) peuvent être utilisés directement dans l'étape iii) ou traiter suivant des techniques bien connues de l'homme du métier
Suivant la nature des dérivés intermédiaires obtenus à l'issu de l'étape ii) le traitement (work-up) sera différent :

a) si le composé est lipophile, le produit est repris dans un solvant organique puis extrait avec une solution aqueuse saturée en hydrogénocarbonate de sodium. La phase aqueuse est ensuite acidifiée puis reprise dans un solvant organique.

b) si le composé hydrophile, il est précipité dans un solvant apolaire, par exemple l'éther diéthylique. Suivant une autre méthode, le composé hydrophile est dissous dans l'eau puis lavé avec un solvant organique; La phase aqueuse est alors lyophilisée.

L'étape iii) consiste à:

- placer le produit obtenu à l'issu de l'étape ii), de préférence 1 équivalent molaire, dans solvant polaire, de préférence anhydre, de préférence l'acétonitrile;
- ajouter au moins un agent de couplage, de préférence 1 équivalent molaire, et/ou un additif pour supprimer l'énantiomérisation lors du couplage, de préférence 1 équivalent molaire

De manière optionnelle, on ajoute une base organique, avantageusement une amine tertiaire, de préférence de la triéthylamine, de préférence 1 équivalent molaire.
Les dérivés de formule (I) obtenus à l'issu de l'étape iii) peuvent être de deux types.

a) Cas des dérivés stables dans les solvants protiques (eau, méthanol):

Le produit sec est repris dans un solvant organique, puis lavé avec de l'eau.
Après traitement habituel de la phase organique, le produit sec est solubilisé dans un solvant

dans lequel les agents de couplage résiduels reste insoluble que l'on élimine par filtration.

b) Cas des dérivés instables dans les solvants protiques (eau, méthanol):

Le produit sec est purifié par chromatographie sur colonne ou par précipitation dans un solvant apolaire, par exemple l'éther diéthylique.

Les produits de formule (I) peuvent être isolés sous forme optiquement pure suivant des méthodes bien connues de l'homme du métier, comme par exemple l'HPLC, y compris en phase inverse.
Un agent de couplage préféré suivant l'invention est choisi parmi DCC (Dicyclohexylcarbodiimide), EDCI (1-(3-dimethylaminopropyl)-3-ethylcarbodiimide, HCl)).
Un additif pour supprimer l'énantiomérisation lors du couplage qui est préféré suivant l'invention est choisi parmi HOBt (1-Hydroxybenzotriazole), NHS (N-Hydroxysuccinimide).
Un agent d'activation in-situ préféré suivant l'invention est HBTU (2(1H-Benzotriazole-yl)-1,1,3,3tétraméthyluronium hexafluorophosphate).
Suivant l'étape i), lorsque l'acide 2-oxothiazolidine 4-carboxylique réagit avec un hydroxyacide ayant la fonction acide protégée, on utilise DCC et NHS.
Suivant l'invention, lorsque l'acide aminé, l'hydroxyacide ou l'halogénoacide de départ, qui est utilisé dans l'étape i) suivant l'invention, présente une chaîne latérale alkyle substitué par au moins un radical -COOH; $-NH_2$; -OH, et/ou -SH, ledit radical est protégé suivant des méthodes classiquement utilisées en synthèse organique et bien connues de l'homme du métier comme par exemple celles décrites dans le document *Protective Groups in Organic Synthesis,* T. W. Greene, P. G. M. Wuts (Wiley Interscience).
Avantageusement, le radical -COOH présent sur la chaîne latérale est protégée par un groupement terbutyl; le radical $-NH_2$ présent sur la chaîne latérale est protégé par un tBoc (terbutyloxycarbonyl); le radical -OH ou -SH présent sur la chaîne latérale est protégée par un radical terbutyl ou tBoc. Ces radicaux -COOH; $-NH_2$; -OH, et/ou -SH seront déprotégés en milieu acide à l'étape ii).
Le procédé de synthèse suivant l'invention peut être mise en oeuvre en utilisant des produits de départ énantiomériquement purs. L'acide 2-oxothiazolidine 4-carboxylique de départ est disponible commercialement sous forme racémique ou sous forme L, elle peut également être obtenue par synthèse sous forme D.
Lorsque les dérivés de formule (I) sont obtenus forme de mélange racémique, les différents diastéréoisomères constitutifs de ce mélange peuvent par exemple être séparés par HPLC préparative suivant des méthodes bien connues de l'homme du métier.
Un autre objet de l'invention se rapporte à une composition, contenant dans un milieu physiologiquement acceptable au moins un dérivé de formule (I) telle que définie précédemment.
La quantité de dérivé(s) de formule (I) utilisable selon l'invention est bien entendue fonction de l'effet recherché et peut donc varier dans une large mesure.
Pour donner un ordre de grandeur, on peut utiliser ce composé en une quantité représentant de 0,0001% à 10% du poids total de la composition, préférentiellement en une quantité représentant de 0,0005% à 5% du poids total

de la composition, plus préférentiellement en une quantité représentant de 0,001% à 1% du poids total de la composition.

Par un milieu physiologiquement acceptable, on entend un milieu compatible avec la peau et éventuellement avec ses phanères (cils, ongles, cheveux) et/ou les muqueuses.

Les compositions suivant l'invention sont adaptées à une application topique cutanée ou à une administration orale.

La composition de l'invention peut se présenter sous toutes formes galéniques imaginables adaptées à une administration topique cutanée ou à une administration par la voie orale

La composition de l'invention peut être à usage cosmétique ou dermatologique. Préférentiellement, la composition de l'invention est à usage cosmétique. Très préférentiellement la composition de l'invention est une composition cosmétique destinée à une administration par la voie orale ou topique cutanée.

La composition de l'invention est une composition cosmétique car elle est destinée maintenir ou à améliorer l'aspect général de la peau d'un individu qui en fait usage.

Pour une administration par la voie orale, la composition de l'invention peut se présenter sous toutes les formes adaptées, particulièrement sous forme d'une solution buvable, d'un sirop, d'un comprimé, d'une gélule ou encore d'une capsule. Préférentiellement, la composition de l'invention se présente sous la forme d'une gélule.

Lorsqu'elle est destinée à une application topique cutanée, la composition peut se présenter sous toutes les formes galéniques normalement utilisées dans le domaine cosmétique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres UV, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des composés de formule (I) selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle ou leur mélange.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme filtres UV, on peut citer les dérivés du benzylidène camphre et les dérivés de benzotriazole.

Avantageusement, les dérivés du benzylidène camphre sont choisis parmi :

- le 3-Benzylidène camphre fabriqué sous le nom « MEXORYL SD» par CHIMEX,
- le 4-Methylbenzylidène camphre vendu sous le nom « EUSOLEX 6300 » par MERCK ,
- le Camphor Benzalkonium Methosulfate fabriqué sous le nom « MEXORYL SO » par CHIMEX,

- le Polyacrylamidomethyl Benzylidene Camphor fabriqué sous le nom « MESORYL SW » par CHIMEX,
- les composés sulfoniques tels que :
  - le Benzylidene Camphor Sulfonic Acid fabriqué sous le nom « MEXORYL SL» par CHIMEX,
  - et plus particulièrement l'acide benzène 1,4-di(3-méthylidène-10-camphosulfonique) encore appelé Terephthalylidene Dicamphor Sulfonic Acid et fabriqué sous le nom « MEXORYL SX » par CHIMEX, et ses différents sels décrits notamment dans les demandes de brevets FR-A-2 528 420 et FR-A- 2 639 347, qui sont des filtres déjà connus en soi (filtres dits à large bande) capables en effet d'absorber les rayons ultraviolets de longueur d'ondes comprises entre 280 et 400 nm, avec des maxima d'absorption compris entre 320 et 400 nm, en particulier aux alentours de 345 nm. Ces filtres répondent à la formule générale (III) suivante :

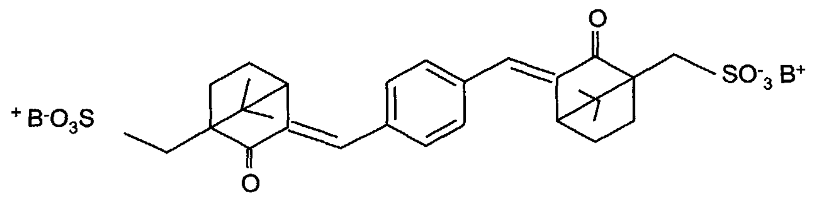

(III)

dans laquelle B désigne un atome d'hydrogène, un métal alcalin ou encore un radical $NH(R)_3+$ dans lequel les radicaux R, qui peuvent être identiques ou différents, désignent un atome d'hydrogène, un radical alkyle ou hydroxyalkyle en $C_1$-$C_4$ ou encore un groupement Mn+/p, Mn+ désignant un cation métallique polyvalent dans lequel p est égal à 2 ou 3 ou 4, Mn+ désignant de préférence un cation métallique choisi parmi $Ca^2+$, $Zn^2+$, $Mg^2+$, $Ba^2+$, $Al^3+$ et $Zr^4+$. Il est bien entendu que les composés de formule (III) ci-dessus peuvent donner lieu à l'isomère "cis-trans" autour d'une ou plusieurs double(s) liaison(s) et que tous les isomères rentrent dans le cadre de la présente invention.

Parmi les dérivés de benzotriazole on peut citer les dérivés silicés décrits dans les brevets EP0 660 701 et EP 0 392 883. Il s'agit en particulier des silanes et/ou des polyorganosiloxanes à fonction benzotriazole comprenant au moins une unité de formule (1) suivante :

$$O_{(3-a)/2}\ Si\ (R_7)_a\ -\ G \qquad (1)$$

dans laquelle :

- $R_7$ représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante :

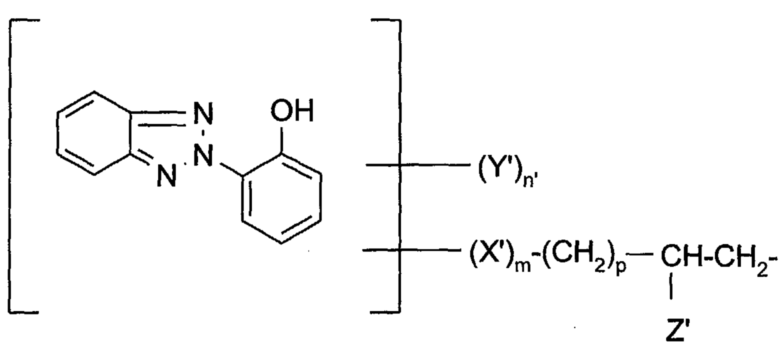

(2)

dans laquelle :

- Y', identiques ou différents, sont choisis parmi les radicaux alkyles en $C_1$-$C_8$, les halogènes et les radicaux alkoxy en $C_1$-$C_4$ étant entendu que, dans ce dernier cas, deux Y' adjacents d'un même noyau aromatique peuvent former ensemble un groupement alkylidène dioxy

dans lequel le groupe alkylidène contient de 1 à 2 atomes de carbone,

- X' représente O ou NH,
- Z' représente l'hydrogène ou un radical alkyle en $C_1$-$C_4$,
- n' est un nombre entier compris entre 0 et 3 inclusivement,
- m est 0 ou 1,
- p représente un nombre entier compris entre 1 et 10, inclusivement.

Avantageusement, le dérivé silicié à fonction benzotriazole répond à l'une des formules (5) ou (6) suivantes :

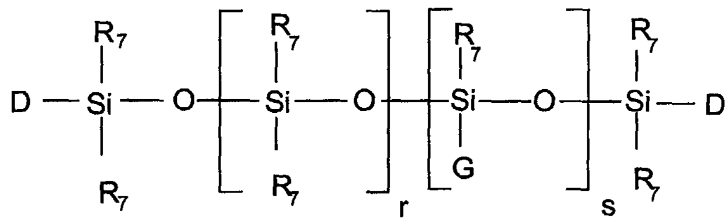

(5)

ou

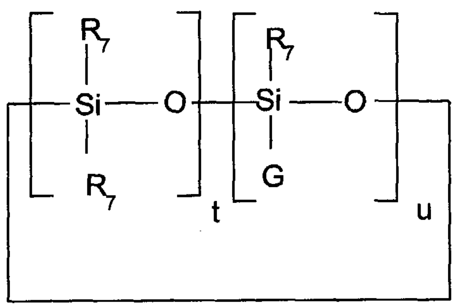

(6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) définie ci-dessus.

Une famille dérivé silicié à fonction benzotriazole préférée répond à la formule (7) suivante :

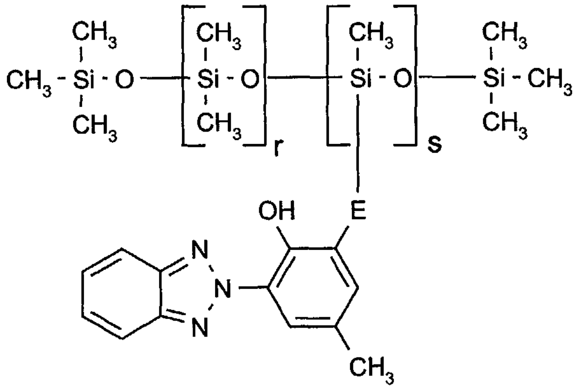

(7)

avec

0≤r≤10,

1≤s≤ 10,

et où E représente le radical divalent :

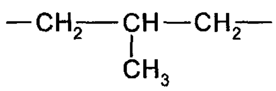

De manière particulièrement préférée le dérivé silicié à fonction benzotriazole est le Drométrizole Trisiloxane de formule suivante :

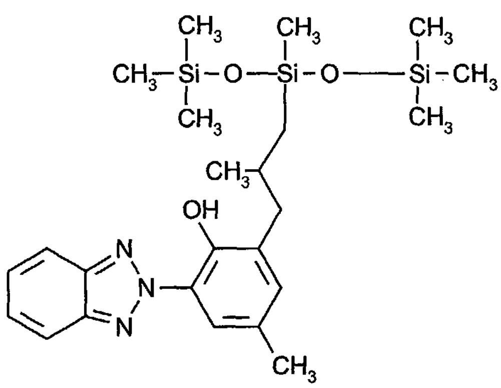

Ce composé est vendu notamment sous le nom « SILATRIZOLE » par RHODIA CHIMIE ,

Les filtres UV sont généralement présents dans les compositions selon l'invention dans des proportions allant de 0,1 à 20% en poids par rapport au poids total de la composition, et de préférence allant de 0,2 à 15% en poids par rapport au poids total de la composition.

Comme actifs, il sera avantageux d'introduire dans la composition utilisée selon l'invention au moins un composé choisi parmi : les agents hydratants ; les agents dépigmentants ou propigmentants ; les agents anti-pollution et/ou anti-radicalaire ; les agents antimicrobiens; les Inhibiteurs de NO-synthase ; les agents stimulant la synthèse de macromolécules dermiques ou épidermiques et/ou empêchant leur dégradation ; les agents stimulant la prolifération des fibroblastes ou des kératinocytes et/ou la différenciation des kératinocytes ; les agents dermo-décontractants ; les agents tenseurs ; les agents apaisants ; les agents agissant sur la microcirculation et/ou les agents agissant sur le métabolisme énergétique des cellules et leurs mélanges.

Des exemples de tels composés additionnels sont donnés ci-dessous.

1. Agents hydratants

Par "agent hydratant", on entend :

- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;

- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;

- soit un composé activant les glandes sébacées tel que les dérivés stéroïdiens (dont la DHEA, ses dérivés 7-oxydés et/ou 17-alkylés et les sapogénines) et la vitamine D et ses dérivés.

Ces composés peuvent représenter de 0,001% à 30%, et de préférence de 0,01 à 20%, du poids total de la composition selon l'invention.

La composition selon la présente invention comprenant les agents hydratants cités ci-dessus est avantageusement destinée à la prévention ou au traitement du dessèchement de la peau et notamment des xéroses.

2. Agent dépigmentant ou pro-pigmentant

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Comme agent pro-pigmentant, on peut citer l'extrait de pimprenelle (Sanguisorba officinalis) commercialisé par la société MARUZEN et les extraits de chrysanthème (Chrysanthemum morifolium).

La composition selon la présente invention comprenant les agents dépigmentants cités ci-dessus est avantageusement destinée à la prévention ou au traitement des hyperpigmentations, en particulier des taches pigmentaires liées au vieillissement de la peau.

De son côté, la composition renfermant les agents pro-pigmentants cités précédemment est de préférence destinée au traitement de la canitie.

3. Agent anti-microbien

Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le farnesol, les phytosphingosines et leurs mélanges.

Les agents antimicrobiens préférés sont le triclosan, le phénoxyéthanol, l'octoxyglycérine, l'octanoylglycine, l'acide 10-hydroxy-2-décanoïque, le caprylyl glycol, le farnesol et l'acide azélaïque.

A titre d'exemple, l'agent antimicrobien peut être utilisé dans la composition selon l'invention en une quantité représentant de 0,1 à 20%, et de préférence de 0,1 à 10%, du poids total de la composition.

La composition renfermant l'agent anti-microbien convient particulièrement bien à une utilisation dans le traitement des peaux grasses à tendance acnéique, l'acné, ou les pellicules du cuir chevelu.

4. Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

5. Inhibiteur de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

La composition selon l'invention comprenant un inhibiteur de NO-synthase tel que défini ci-dessus peut avantageusement être utilisée pour prévenir ou traiter les signes du vieillissement cutané et/ou les peaux sensibles.

6. Agent stimulant la synthèse de macromolécules dermiques ou épidermiques et/ou empêchant leur dégradation

Parmi les actifs stimulant les macromolécules du derme ou empêchant leur dégradation, on peut citer ceux qui agissent :

- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.

- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;

- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;

- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;

- soit sur l'inhibition des métalloprotéinases (MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;

- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.
La composition selon l'invention renfermant un ou plusieurs des composés ci-dessus convient particulièrement bien à une utilisation dans la prévention ou le traitement des signes cutanés du vieillissement, en particulier de la perte de fermeté et/ou d'élasticité de la peau.

7. Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.
Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ;

les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine® ; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl® ; et les lignanes tels que le sécoisolaricirésinol..

La composition selon l'invention comprenant ces composés est préférentiellement destinée à être utilisée pour prévenir ou traiter les signes cutanés du vieillissement.

8. Agent dermo-décontractant

Les agents dermo-décontractants utilisables dans la composition selon l'invention comprennent l'alvérine et ses sels, le gluconate de manganèse, le Diazepam, l'hexapeptide argireline R commercialisé par la société LIPOTEC, certaines amines secondaires et tertiaires carbonylées, l'adénosine, ainsi que les sapogénines et les extraits naturels, en particulier de Wild Yam, en contenant.

La composition selon l'invention comprenant ces composés est préférentiellement destinée à être utilisée pour prévenir ou traiter les signes cutanés du vieillissement et en particulier les rides.

9. Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :

(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,

(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,

(3) les protéines et hydrolysats de protéines végétales, en particulier de maïs, de seigle, de froment, de sarrasin, de sésame, d'épautre, de pois, de fève, de lentille, de soja et de lupin, (3) les silicates mixtes, notamment les phyllosilicates et en particulier les Laponites,

(4) les microparticules de cire, choisies par exemple parmi les cires de Carnauba, de Candelila ou d'Alfa,

(5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.

Les compositions selon l'invention comprenant les agents tenseurs ci-dessus sont avantageusement destinées au traitement des signes cutanés du vieillissement, en particulier des rides et ridules.

10. Agents apaisants

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en

oméga 3 telles que les huiles de rosier musca, de cassis, d'ecchium, de poisson, des extraits de plancton, la capryloyl glycine, le Seppicalm VG (sodium palmitoylproline et nymphéa alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa moniera, les phytostérols, la cortisone, l'hydrocortisone, l'indométhacine et la beta méthasone.

11. Agents agissant sur la microcirculation

Les actifs agissant sur la microcirculation (vasoprotecteur ou vasodilatateur) peuvent être choisis parmi les flavonoïdes, les ruscogénines, les esculosides, l'escine extraite du marron d'Inde, les nicotinates, l'héperidine méthyl chalcone, les huiles essentielles de lavande ou de romarin, les extraits de Ammi Visnaga.

La quantité de ces actifs peut varier dans une large mesure. De manière générale, ces actifs sont présents en une concentration allant de 0,01 à 15 % et de préférence de 0,05 à 10 % en poids par rapport au poids total de la composition.

12. Agents agissant sur le métabolisme énergétique des cellules

Les actifs concernés sont ceux qui agissent sur le métabolisme énergétique cutané tel que, par exemple, et de façon non limitative, la synthèse d'ATP, ceux qui sont interviennent sur la chaine respiratoire de la cellule ou sur les réserves énergétiques. On peut citer le Coenzyme Q10 (ubiquinone), le cytochrome C, la créatine ou encore la phosphocréatine.

L'invention a encore pour objet un procédé de traitement cosmétique de la peau par administration orale ou application topique cutanée d'une composition cosmétique telle que définie précédemment pour maintenir ou améliorer l'aspect général de la peau d'un individu qui en fait usage.

L'invention est illustrée plus en détail dans les exemples suivants. Ces exemples ne sauraient limiter en aucune façon la portée de l'invention.

**EXEMPLE 1 : Procédé général de synthèse des dérivés de formule (I)**

Première étape avec un acide aminé ou un hydroxyacide comme produit de départ

Après séchage sous vide-azote et sous azote, on place 4 g d'acide L-2-oxothiazolidine 4-carboxylique (Fournisseur Aldrich) (0.0271 mol, 1 équivalent) dans un tricol de 500mL. On ajoute 200 mL d'acétonitrile anhydre et on chauffe jusqu'à solubilisation complète. On ajoute en une fois 3.15 g de N-Hydroxysuccinimide 98 % (NHS, 0.0271 mol, 1 équivalent). On refroidit à 0°C avant d'ajouter goutte à goutte une solution de 5.61 g de Dicyclohexilcarbodiimide 98% (DCC, 0.0271 mol, 1 équivalent) solubilisés dans 70 mL d'acétonitrile anhydre.

On laisse agiter à 0°C pendant une heure puis une heure à température ambiante. On ajoute 1 équivalent d'ester terbutylique d'acide aminé chlorhydraté ou d'ester terbutylique d'hydroxyacide en une portion et 7.58 mL de triéthylamine (TEA, 0.0544 mol, 2 équivalents).

Lorsque l'ester terbutylique de l'acide aminé ou de l'hydroxyacide n'est pas un chlorhydrate, on n'utilise qu'un équivalent de triéthylamine.

On agite pendant 15 h.

Le précipité blanc obtenu est filtré et le filtrat est évaporé sous pression réduite (pression de 200 mbar, Température de 40°C). L'huile obtenue est reprise dans 200 mL d'acétate d'éthyle (ou dichlorométhane) et lavé par 3x100 mL d'eau et 100mL de solution de chlorure de sodium saturée. La phase organique est séchée sur sulfate de sodium, filtrée et évaporée sous pression réduite (p=220 mbar, T=40°C).

Le produit pur est obtenu par chromatographie colonne (Gel de silice 60 0.04-0.063 mm), éluant : dichlorométhane/méthanol : 98/2.

Première étape avec un halogénoacide comme produit de départ

Une solution de 20 mmol d'acide L-2-oxothiazolidine 4-carboxylique et de 10.2 mmol de $K_2CO_3$ en solution dans la DMF sont portés à 80° puis 22.4mmol d'halogénoacide (protégé sous forme d'ester terbutylique) sont additionnés. Le milieu réactionnel est maintenu sous agitation et à 80°C pendant 3h. Après retour à température ambiante, le milieu réactionnel est dilué par 300ml d'acétate d'éthyle puis lavé par 3 fois avec 100ml d'eau puis encore une fois par 10ml d'une solution saturée en NaCl. La phase organique est séchée sur $Na_2SO_4$, filtrée puis concentré sous pression réduite (p=220 mbar, T=40°C).

Deuxième étape

Dans un tricol de 100 mL, on place 1 équivalent du produit obtenu à l'issu de la première étape (0.01764 mol, 1 équivalent) dans 65 mL de 1,2 dichloroéthane. On ajoute 6,75 mL d'acide trifluoroacétique (TFA, 0.0882 mol, 5 équivalents) et 2,82 mL de triéthylsilane (0.01764 mol, 1 équivalent). On chauffe à 50°C pendant 15 h.
On évapore le 1,2 dichloroéthane sous pression réduite (p=200 mbar, T=40°C).

Traitement :

a) Si le composé est lipophile : l'huile obtenue est reprise dans 100 mL d'acétate d'éthyle, extraite par 2x50 mL de solution d'hydrogénocarbonate de sodium saturée, puis 2x 50 mL d'eau. La phase aqueuse est acidifiée jusqu'à pH=2 par une solution d'acide chlorhydrique 6N puis extraite par 6x100 mL d'acétate d'éthyle. Les phases organiques réunies sont séchées sur sulfate de sodium, filtrées, puis évaporées sous pression réduite (P=220 mbar, 40°C).

b) Si le composé est hydrophile : l'huile obtenue est reprise dans 100 mL d'eau et lavée par 2x40 mL de dichlorométhane puis lyophilisée (P=10-2 mbar, T=-50°C).

Troisième étape

Après séchage sous vide-argon, on charge dans un tricol de 500 mL 1 équivalent du produit obtenu à l'issu de la deuxième étape (0.01385 mol) dans 100mL d'acétonitrile anhydre. On ajoute 1.606g de N-hydroxysuccinimide 98% (NHS, 0.01385 mol, 1 équivalent) et 2.657 g de 1-(3-dimethylaminopropyl)-3-ethylcarbodiimide, HCl (EDCI, 0.01385 mol, 1 équivalent). On laisse agiter à température ambiante pendant 2 heures. On dilue la solution par 400 mL d'acétonitrile anhydre, ajoute 1.93 mL de triéthylamine (TEA, 0.01385 mol, 1 équivalent) et laisse agiter pendant trois heures à température ambiante.
Le mélange réactionnel est évaporé sous pression réduite (p=200 mbar)

Traitement :

a) Cas des dérivés stables dans l'eau:
Reprise du brut évaporé dans 100 mL de dichlorométhane et lavage par 3x20 mL d'eau. La phase organique est séchée sur sulfate de sodium, filtrée et évaporée sous pression réduite (p=950 mbar, T=40°C). On obtient un solide blanc qui est repris dans 8 mL d'acétone. On filtre le précipité et on lave par 3x2 mL d'acétone. Cette opération est répétée 2 fois (élimination totale de N-hydroxysuccinimide). Le produit pur est obtenu après séchage sous pression réduite (P=$10^{-1}$ mbar, TA).

b) Cas des dérivés instables dans l'eau.
Purification du brut évaporé sur chromatographie colonne (flash master personnal, colonne BP SUP), éluant cyclohexane/acétone 8/2. Le solide obtenu est repris dans 2 mL d'acétone. On ajoute de l'éther diéthylique progressivement jusqu'à précipitation. Le produit pur est isolé par précipitation après séchage sous pression réduite (P=$10^{-1}$ mbar, Température Ambiante).

**EXEMPLE 2: Composés obtenus suivant le procédé de synthèse de l'exemple 1**

**Composé n°1:** 6-Phenyl-tétrahydro-thiazolo[3,4-a]pyrazine-3,5,8-trione

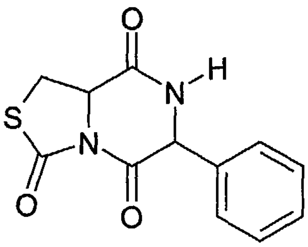

Mélange de diastéréoisomères A/B : 5/1
RMN $^1$H (400MHz ; DMSO) δ ppm 3.58 (m, 4H) ; 4.93 (dd, 1H, J=10.98 Hz et 7.69 Hz, A) ; 5.14 (dd, 1H, J=10.43 Hz et 8.23 Hz, B) ; 5.21 (s, 1H, A) ; 5.37 (s, 1H, B) ; 7.40 (m, 10H) ; 8.86 (s, 1 H, B) ; 9.18 (s, 1 H, A).
La masse du produit est confirmée par le spectre de masse suivant :
MS : m/z=285 (M+Na, 100%), 261 ( MH-, 100%).

**Composé n°2:** Tétrahydro-thiazolo[3,4-a]pyrazine-3,5,8-trione

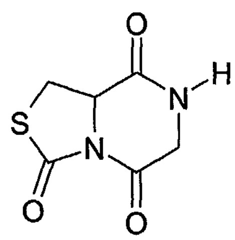

RMN $^1$H (400MHz ; DMSO) δ ppm: 3.53 (d, 2H, H7, J=9.51 Hz) ; 3.72 (dd, 1H, H4, J=4.66 et 17.2 Hz) ; 4.17 (d, 1 H, H4, J=17.2 Hz) ; 5.00 (t, 1 H, H6a, J=9.51 Hz) ; 8.52 (s, 1H, NH).
La masse du produit est confirmée par le spectre de masse suivant :
MS : m/z=186.91 (MH+, 10%), 208.91 (M+Na, 6%), 185.08 (M-, 2%).

**Composé n°3:** 6-Benzyl-tétrahydro-thiazolo[3,4-a]pyrazine-3,5,8-trione

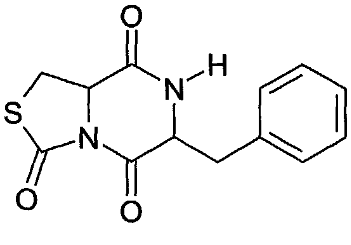

RMN $^1$H (400MHz ; DMSO) δ ppm: 3.01 (dd, 1H, H1', J= 5.49 et 13.54 Hz) ; 3.14 (dd, 1H, H', J= 6.86 et 13.72 Hz) ; 3.40 (m, 2H, H7) ; 4.06 (dd, 1H, H6a, J=10.98 et 7.68 Hz) ; 4.19 (dd, 1H, H4, J=10.71 et 5.58 Hz) ; 7.20 (m, 2H, C6H5) ; 7.33 (m, 3H, C6H5) ; 8.57 (s, 1H, NH).

La masse du produit est confirmée par le spectre de masse suivant :
MS : m/z=276.98 (MH+, 14%), 298.95 (M+Na, 100%), 275.03 (M-, 100%).

**Composé n°4:** Hexahydro-pyrolo[1,2-a]thiazolo[3,4-d]pyrazine-3,5,10-trione

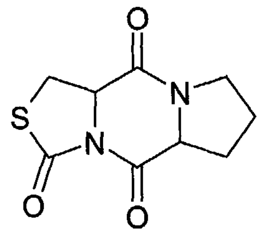

RMN $^1$H (400MHz ; DMSO) δ ppm: 1.87 (m, 2H, H5) ; 2.05 (m, 1H, H4) ; 2.18 (m, 1H, H4) ; 3.41 (m, 2H, H6) ; 3.54 (dd, 2H, H8, J=4.02 et 9.33 Hz) ; 4.49 (t, 1H, H3a, J=7.96 Hz) ; 5.13 (t, 1H, H7a, J=9.24 Hz).
La masse du produit est confirmée par le spectre suivant :
MS : m/z=226.96 (MH+, 15%), 248.95 (M+Na, 100%), 225.00 ( M-, 100%).

**Composé n°5:** 1H-[1,3]thiazolo[4,3-c]oxazine-3,5,8[6H,8aH]-trione

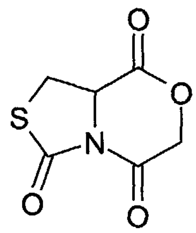

RMN $^1$H (400MHz ; DMSO) δ ppm
3.64 (m, 1H, H3a); 3.94 (m, 1H, H3b); 5.09 (d, 1H, H6a); 5.35 (m, 1H, H4); 5.53 (d, 1H, H6b) RMN $^{13}$C (400MHz ; DMSO) δ ppm
29.28 (C3); 57.49 (C4); 64.48 (C6); 164.98 (C7); 167.98 (C5); 172 (C1)

**EXEMPLE 3 : Méthode d'évaluation de la photo-protection active des dérivés de l'invention vis-à-vis d'un stress oxydatif UVA induit sur kératinocytes.**

La technique d'évaluation de la photo-protection est réalisée suivant une méthode bien connue (J. of Photochemistry and Photobiology B : Biology 57 (2000) 102-112 TOBI et al : Glutathione modulates the level of free radicals produced in UVA irradiated cells). Cette technique utilise une sonde fluorescente, marqueur du stress oxydatif global intracellulaire, la 2'-7'-Dichlorofluorescine Diacétate (DCFH-DA).

PRINCIPE

L'utilisation de la DCFH-DA comme marqueur du stress oxydatif repose sur ses propriétés physico-chimiques. Il s'agit d'une molécule apolaire et non ionique capable de diffuser à travers les membranes cellulaires. Une fois à l'intérieur de la cellule, la DCFH-DA va être hydrolysée par des estérases intracellulaires en un composé non fluorescent : la DCFH ou 2,7-dichlorofluorescine. En présence d'espèces activées de l'oxygène, la DCFH est rapidement oxydée en un composé hautement fluorescent: la DCF ou 2,7-dichlorofluorescéine.

MODE OPERATOIRE

1. Traitement des kératinocytes par un composé de formule (I)
A confluence, les kératinocytes sont incubés en présence d'un composé de formule (I) pendant 24 heures à 37°C, 5% de $CO_2$, dans le milieu de culture, selon un effet dose (3 concentrations).

2. Incorporation de la DCFH-DA
Les kératinocytes, prétraités avec un composé de formule (I) sont rincés puis incubés en présence de DCFH-DA à l'obscurité.

3. Exposition aux UVA
A l'issue de cette incubation, la solution de DCFH-DA est éliminée, les cellules sont ensuite exposées aux UVA. Remarque : une plaque témoin non exposée est conservée à l'obscurité, à température ambiante.

4. Mesure de la fluorescence
La fluorescence de la DCF est évaluée immédiatement après l'exposition aux UVA, par spectrofluorimétrie (excitation : 480nm ; émission : 530nm).

5. Résultats :
Les résultats sont exprimés en % de fluorescence par rapport aux cellules témoins exposées aux UVA :

| **Composé n°2 [mM]** | TEST 1 | TEST 2 | **Moyenne** | Ecart-type |
|---|---|---|---|---|
| **0** | 100 | 100 | **100** | 0 |
| **0.05** | 98 | 86 | **92** | 8.5 |
| **0.5** | 79 | 74 | **76.5** | 3.5 |
| **5** | 69 | 58 | **63.5** | 7.8 |

L'effet photo-protecteur du composé n°2 a été évalué à 0.05, 0.5 et 5mM. Les résulats révélent une efficacité protectrice du composé n°2 vis-à-vis du stress oxydatif UVA induit selon un effet dose : l'efficacité optimale est obtenue à 5mM (dimunition de la fluorescence d'environ 36%).
Il apparaît clairement que les dérivés de formule (I) selon l'invention induisent une photo-protection active de la peau par rapport au témoin non traité.

**EXEMPLE 4 Compositions**

Tablette de 0.2g obtenue après compactage :

- Composé n° 1 0,001g
- Amidon 0,114g
- Phosphate de Calcium 0,020g
- Lactose 0,060g
- Stéarate de Magnésium 0,005g

Les tablettes obtenues sont adaptées à une administration par voie orale.

Suspension par voie orale

- Composé n° 1 0,001 g
- Glycérol 0,500g
- Sorbitol 0,500g
- Saccharinate de sodium 0,010g
- Para-hydroxybenzoate de méthyl 0,040g
- Arôme qsp
- complément à 5ml d'eau purifiée

Crème pour le visage

- Composé n° 3 1,00%
- Stéarate de sodium 3,00%
- Huile de vaseline 6,00%
- Alkyl paraben 0,05%
- Sorbate de potassium 10,00%
- Alcool stéarylique 1,00%
- Parfum 1,00%
- Eau qsp 100,00%

Crème pour le corps

- Composé n° 3 0,5%
- Huile de jojoba 13,0%
- Cire de sipol 6,0%
- Palmitate d'isopropyle 2,0%
- Glycérol 15,0%
- Alkyl paraben 0,5%
- Parfum 1,0%
- Eau qsp 100,0%

Crème de soin solaire

- Composé n° 3 1%
- Polyéthylène glycol 50 oxyéthyléné 3%
- Mono diglycéryl stéarate 3%
- Huile de vaseline 24%
- Alcool cétylique 5%
- Eau qsp 100%

Crème de soin solaire pour le corps

- Composé n° 3 0,5%
- Cire de sipol 6,0%
- Glycéryl monostéarate 1,5%
- Stéarate de sodium 0,8%
- Huile de vaseline 6,0%

- Palmitate d'isopropyle 2,0%
- Glycérol 15,0%
- Parfum 0,3%
- Eau qsp 100,0%

Crème de soin

- Composé n°3 0,1%
- Huile de jojoba 13,00%
- Alkyl paraben 0,05%
- Sorbate de potassium 0,30%
- Cyclopenta diméthylsiloxane 10,00%
- Alcool stéarylique 1,00%
- Acide stéarique 4,00%
- Stéarate de polyéthylène glycol 3,00%
- Vitamine E 1,00%
- Glycérol 3,00%
- Eau qsp 100,00%

Crème Solaire

| | |
|---|---|
| CETEARYL ALCOHOL (and) CETEARETH-30 vendue par la société Cognis sous la dénomination SINNOWAX AO | 7% |
| GLYCERYLSTEARATE vendue par la Stéarinerie Dubois sous la dénomination STEARATE DE GLYCEROL 50/50 | 2% |
| CETYL ALCOHOL vendue par la société Cognis sous la dénomination LANETTE 16 | 1.5% |
| DIMETHICONE vendue par la société Dow Corning sous la dénomination DC 200 FLUID 350 CS | 1.5% |
| MINERAL OIL vendue par la société Esso sous la dénomination MARCOL 82 | 15% |
| DROMETRIZOLE TRISILOXANE fabriqué par la société Chimex sous le nom MEXORYL XL | 4% |
| TEREPHTALYLIDENE DICAMPHOR SULFONIC ACID fabriqué par la société Chimex sous le nom MEXORYL SX | 1.5% |
| TRIETHANOLAMINE vendue par la société BASF sous le nom TRIETHANOLAMINE CARE | 0.26% |
| GLYCERIN vendue par la société Uniquema sous la dénomination PRICERINE 9091 | 20% |
| COMPOSE n°2 6-Benzyl-Tétrahydro-thiazolo[3,4-a]pyrazine-3,5,8-trione | 0.1% |
| CONSERVATEUR | Qsp |
| EAU | Qsp 100g |

Tablette de 0.2g obtenue après compactage :

- Composé n° 2 0 ,001g
- Amidon 0,114g
- Phosphate de Calcium 0,020g
- Lactose 0,060g
- Stéarate de Magnésium 0,005g

Les tablettes obtenues sont adaptées à une administration par voie orale.

Suspension par voie orale

- Composé n° 2 0,001g
- Glycérol 0,500g
- Sorbitol 0,500g
- Saccharinate de sodium 0,010g
- Para-hydroxybenzoate de méthyl 0,040g
- Arôme qsp
- complément à 5ml d'eau purifiée

Crème pour le visage

- Composé n° 2 1,00%
- Stéarate de sodium 3,00%
- Huile de vaseline 6,00%
- Alkyl paraben 0,05%
- Sorbate de potassium 10,00%
- Alcool stéarylique 1,00%
- Parfum 1,00%
- Eau qsp 100,00%

Crème pour le corps

- Composé n° 2 0,5%
- Huile de jojoba 13,0%
- Cire de sipol 6,0%
- Palmitate d'isopropyle 2,0%
- Glycérol 15,0%
- Alkyl paraben 0,5%
- Parfum 1,0%
- Eau qsp 100,0%

Crème de soin solaire

- Composé n° 2 1 %
- Polyéthylène glycol 50 oxyéthyléné 3%
- Mono diglycéryl stéarate 3%
- Huile de vaseline 24%
- Alcool cétylique 5%
- Eau qsp 100%

Crème de soin solaire pour le corps

- Composé n° 2 0,5%
- Cire de sipol 6,0%
- Glycéryl monostéarate 1,5%
- Stéarate de sodium 0,8%
- Huile de vaseline 6,0%

- Palmitate d'isopropyle 2,0%
- Glycérol 15,0%
- Parfum 0,3%
- Eau qsp 100,0%

Crème de soin

- Composé n° 2 0,1%
- Huile de jojoba 13,00%
- Alkyl paraben 0,05%
- Sorbate de potassium 0,30%
- Cyclopenta diméthylsiloxane 10,00%
- Alcool stéarylique 1,00%
- Acide stéarique 4,00%
- Stéarate de polyéthylène glycol 3,00%
- Vitamine E 1,00%
- Glycérol 3,00%
- Eau qsp 100,00%

Crème Solaire

| | |
|---|---|
| CETEARYL ALCOHOL (and) CETEARETH-30 vendue par la société Cognis sous la dénomination SINNOWAX AO | 7% |
| GLYCERYLSTEARATE vendue par la Stéarinerie Dubois sous la dénomination STEARATE DE GLYCEROL 50/50 | 2% |
| CETYL ALCOHOL vendue par la société Cognis sous la dénomination LANETTE 16 | 1.5% |
| DIMETHICONE vendue par la société Dow Corning sous la dénomination DC 200 FLUID 350 CS | 1.5% |
| MINERAL OIL vendue par la société Esso sous la dénomination MARCOL 82 | 15% |
| DROMETRIZOLE TRISILOXANE fabriqué par la société Chimex sous le nom MEXORYL XL | 4% |
| TEREPHTALYLIDENE DICAMPHOR SULFONIC ACID fabriqué par la société Chimex sous le nom MEXORYL SX | 1.5% |
| TRIETHANOLAMINE vendue par la société BASF sous le nom TRIETHANOLAMINE CARE | 0.26% |
| GLYCERIN vendue par la société Uniquema sous la dénomination PRICERINE 9091 | 20% |
| COMPOSE n°2 Tétrahydro-thiazolo[3,4-a]pyrazine-3,5,8-trione | 0.1% |
| CONSERVATEUR | Qsp |
| EAU | Qsp 100g |

**Revendications**

1. Dérivés de formule (1) suivante:

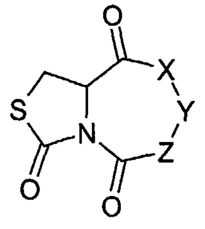

(I)

dans laquelle,

- X représente un atome d'oxygène ou un radical NR";
  - R" représente un radical alkyle ayant de 1 à 12 atomes de carbone, linéaire, cyclique ou ramifié, éventuellement substitué ;
- Y représente un groupement $(CH_2)_n$ ;
- Z représente un groupement (CRR') ;
  - n est un nombre entier choisi parmi 0,1, 2 ou 3;
  - R et R', identiques ou différents représentent :
    - un atome d'hydrogène;
    - un radical $-Si-(CH_3)_3$ ; $-Si(CH_3)_2-OH$ ; $-Si-(CH_3)_2-OEt$, $-Si-(CH_3)_2-O-CH_2-Ph$ ; $-Si-(iPr)_2-OEt$ ;
    - un halogène;
    - un radical alkyle tel que défini précédemment
    - un radical aryle, éventuellement substitué;
    - un radical arylalkyle, éventuellement substitué;

  étant entendu que lorsque X représente un radical NR", R" et R pris ensemble peuvent former avec les 2 atomes les portant, un hétérocycle ayant 4, 5 ou 6 atomes de carbone;
  ou,
- Y et Z représentent chacun un atome de carbone et forment ensemble un cycle ou un hétérocycle aromatique ayant 5, 6 ou 7 atomes de carbone, le dit cycle ou hétérocycle étant éventuellement substitué;

ses isomères optiques et/ou géométriques, seuls ou en mélange en toutes proportions, ainsi que ses sels physiologiquement acceptables.

2. Dérivés de formule (I) suivant la revendication précédente, **caractérisé en ce que** n est choisi parmi 0 ou 1.

3. Dérivés de formule (I) suivant l'une quelconque des revendications précédentes, **caractérisé en ce que** le radical aryle est un cycle ou un hétérocycle aromatique ayant 5 ou 6 chaînons, avantageusement le radical phényle.

4. Dérivés de formule (I) suivant l'une quelconque des revendications 1 à 2, **caractérisé en ce que** le radical arylalkyle est un radical de 6 à 12 atomes de carbone, dans lequel la partie aryle est un cycle ou un hétérocycle aromatique ayant 5 ou 6 chaînons ou un hétérocycle aromatique ayant 5 ou 6 chaînons.

5. Dérivés de formule (I) suivant l'une quelconque des revendications 1 à 2, **caractérisé en ce que** le radical aryle substitué ou le radical arylalkyle substitué est substitué par au moins un groupement choisi parmi un groupement hydroxy (-OH), un groupement cyano (-CN), un groupement trifluorométhyle ($-CF_3$), un radical méthoxy ($-OCH_3$) ou un atome d'halogène choisi parmi le chlore, le brome, le fluor, l'iode.

6. Dérivés de formule (I) suivant la revendication précédente, **caractérisé en ce que** le radical aryle substitué est un radical phényle substitué par groupement un radical alkyle, un groupement hydroxy ou un radical méthoxy.

7. Dérivés de formule (I) suivant la revendication 6, **caractérisé en ce que** le radical arylalkyle substitué est un radical benzyle substitué par au moins un groupement choisi parmi un radical alkyle, un groupement hydroxy ou un radical méthoxy.

8. Dérivé de formule (I) suivant l'une quelconque des revendications précédentes, **caractérisé en ce que** le radical alkyle est en $C_1$-$C_4$, de préférence choisi parmi les radicaux méthyle, éthyle, propyle, isopropyle, butyle, isobutyle ou tertiobutyle.

9. Dérivé de formule (I) suivant l'une quelconque des revendications précédentes, **caractérisé en ce que** :

   - X représente un atome d'oxygène ou un radical NR";
   - Z représente un groupement (CRR') et n est égal à 0;

- R et R", identiques ou différents, représentent un atome d'hydrogène, un radical alkyle tel que défini précédemment, ou un cycloakyle;
- R' représente un atome d'hydrogène, un radical phényle, un radical benzyle ou la chaîne latérale d'un acide aminé, de préférence choisie parmi celle de l'alanine, l'isoleucine, la leucine, la méthionine, la phénylalanine, le tryptophane, la valine, l'asparagine, la cystéine, la glutamine, le glycocolle, la sérine, la thréonine, l'acide aspartique, l'acide glutamique, l'arginine, l'histidine, la lysine;

étant entendu que R" et R pris ensemble peuvent former avec les 2 atomes les portant, la chaîne latérale de la proline,

ou,

- Y et Z représentent chacun un atome de carbone et forment ensemble un cycle aromatique éventuellement substitué, ayant 6 atomes de carbone.

**10.** Dérivés de formule (1) suivant l'une quelconque des revendications précédentes choisis parmi:

- Tetrahydro-thiazolo[3,4-a]pyrazine-3,5,8-trione ;
- 6-Methyltetrahydro[1,3]thiazolo[3,4a]pyrazine-3,5,8trione ;
- 6-Ethyltetrahydro[1,3]thiazolo[3,4a]pyrazine-3,5,8trione ;
- 6-Propyltetrahydro[1 ,3]thiazolo[3,4a]pyrazine-3,5,8trione ;
- 6-iso-Propyltetrahydro[1 ,3]thiazolo[3,4a]pyrazine-3,5,8trione ;
- 6-Propyltetrahydro[1,3]thiazolo[3,4a]pyrazine-3,5,8-trione ;
- 6-[1-Methylpropyl]tetrahydro[1,3]thiazolo[3,4-a]pyrazine-3,5,8-trione ;
- 6-isobutyltétrahydro[1,3]thiazolo[3,4a]pyrazine-3,5,8-trione ;
- 6-Phenyltetrahydro[1,3]thiazolo[3,4a]pyrazine-3,5,8-trione ;
- 6-Benzyltetrahydro[1,3]thiazolo[3,4a]pyrazine-3,5,8-trione ;
- 6-(Methylsulfanyl)ethyl)tetrahydro[1,3]thiazolo[3,4a]pyrazine-3,5,8-trione ;
- 6-(Ethylsulfanyl)methyl)tetrahydro[1,3]thiazolo[3,4a]pyrazine-3,5,8-trione ;
- 7-Methyltetrahydrotetra[1,3]thiazolo[3,4-a]pyrazine-3,5,8-trione ;
- 7-Ethyltetrahydrotetra[1,3]thiazolo[3,4-a]pyrazine-3,5,8-trione ;
- Tetrahydro-1H,5H-pyrrolo[1,2a][1,3]thiazolo[3,4d]pyrazine,3,5,10(10aH)-trione ;
- 3-(3,5,8-Trioxo-hexahydro-thiazolo[3,4-a]pyrazin-6-yl)-propionic acid ;
- 6-Mercaptomethyl-tetrahydro-thiazolo[3,4-a]pyrazine-3,5,8-trione ;
- 7-Methyltétrahydro-thiazolo[3,4-a]pyrazine-3,5,8-trione ;
- 6,7-Dimethyltetrahydro[1,3]thiazolo[3,4a]pyrazine-3,5,8trione ;
- 6-Ethyl,7-methyltetrahydro[1,3]thiazolo[3,4a]pyrazine-3,5,8trione ;
- 6-Propyl,7-methyltetrahydro[1,3]thiazolo[3,4a]pyrazine-3,5,8trione ;
- 6-iso-Propyl,7-methyltetrahydro[1,3]thiazolo[3,4a]pyrazine-3,5,8trione ;
- 6-Propyl,7-methyltetrahydro[1,3]thiazolo[3,4a]pyrazine-3,5,8-trione ;
- 6-[1-Methylpropyl],7-methyltetrahydro[1,3]thiazolo[3,4-a]pyrazine-3,5,8-trione ;
- 6-iso-Butyl,7-methyltétrahydro[1,3]thiazolo[3,4a]pyrazine-3,5,8-trione ;
- 6-Phenyl,7-methyltetrahydro[1,3]thiazolo[3,4a]pyrazine-3,5,8-trione ;
- 6-Benzyl,7-methyltetrahydro[1,3]thiazolo[3,4a]pyrazine-3,5,8-trione ;
- 6-(Methylsulfanyl)ethyl),7-methyltetrahydro[1,3]thiazolo[3,4a]pyrazine-3,5,8-trione;
- 6-(Ethylsulfanyl)methyl),7-methytetrahydro[1,3]thiazolo[3,4a]pyrazine-3,5,8-trione;
- 3-(3,5,8-Trioxo-hexahydro-7-methyl-thiazolo[3,4-a]pyrazin-6-yl)-propionic acid ;
- 6-Mercaptomethyl-7-methyl-tetrahydro-thiazolo[3,4-a]pyrazine-3,5,8-trione ;
- 1H-[1,3]thiazolo[4,3-c]oxazine-3,5,8[6H,8aH]-trione ;
- 1,10a-Dihydro-9-oxa-2-thia-3a-aza-benzo[f]azulène-3,4,10-trione ;
- 6-Octanoyl-1,10a-dihydro-9-oxa-2-thia-3a-aza-benzo[f]azulène-3,4,10-trione ;
- (6-Carboxymethyl-3,5,8-trioxo-tetrahydro-thiazolo[4,3-c][1,4]oxazin-6-yl)-acetic acid.

**11.** Procédé de fabrication des dérivés de formule (I) telle que définie dans l'une quelconque des revendications précédentes, à partir de l'acide L-2-oxothiazolidine 4-carboxylique, **caractérisé en ce qu'**il comprend les étapes suivantes:

i) Réaction de couplage, en présence d'une base entre l'acide 2-oxothiazolidine 4-carboxylique et un acide aminé, un hydroxyacide ou un halogénoacide dont la fonction acide est protégée;
ii) Réaction de déprotection par un agent acide de la fonction acide protégée du produit obtenu à l'issu de

l'étape i);
iii) Réaction de cyclisation intramoléculaire de type peptidique;

étant entendu que l'étape iii) est mise en oeuvre si la cyclisation intramoléculaire ne se produit spontanément à l'étape ii).

**12.** Composition, contenant dans un milieu physiologiquement acceptable au moins un dérivé de formule (I) telle que définie dans l'une quelconque des revendications 1 à 10.

**13.** Composition selon la revendication précédente, **caractérisée en ce que** ledit dérivé de formule (I) représente 0,0001% à 10% du poids total de la composition.

**14.** Composition selon l'une quelconque des revendications 12 à 13, **caractérisée en ce que** ladite composition renferme en outre au moins un adjuvant choisi parmi les gélifiants hydrophiles ou lipophiles, les actifs hydrophiles ou lipophiles, les conservateurs, les antioxydants, les solvants, les parfums, les charges, les filtres UV, les pigments, les absorbeurs d'odeur et les matières colorantes.

**15.** Composition suivant la revendication précédente, **caractérisée en ce que** les filtres UV sont choisis parmi les dérivés du benzylidène camphre et les dérivés de benzotriazole.

**16.** Composition suivant la revendication précédente, **caractérisée en ce que** les dérivés du benzylidène camphre sont choisis parmi

- le 3-Benzylidène camphre ;
- le 4-Methylbenzylidene camphre ;
- le Camphor Benzalkonium Methosulfate,
- le Polyacrylamidomethyl Benzylidene Camphor,
- les composés sulfoniques tels que :
    - le Benzylidene Camphor Sulfonic Acid,
    - l'acide benzène 1,4-di(3-méthylidène-10-camphosulfonique) et ses différents sels de formule générale (III) suivante :

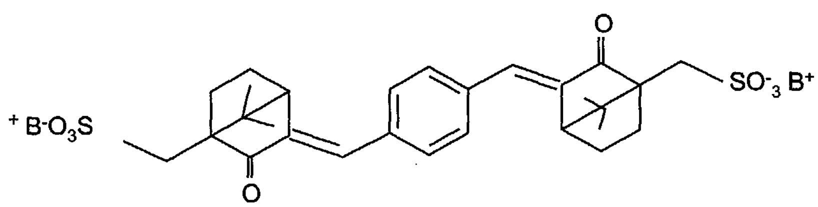

(III)

dans laquelle B désigne un atome d'hydrogène, un métal alcalin ou encore un radical $NH(R)_3+$ dans lequel les radicaux R, qui peuvent être identiques ou différents, désignent un atome d'hydrogène, un radical alkyle ou hydroxyalkyle en $C_1$-$C_4$ ou encore un groupement Mn+/p, Mn+ désignant un cation métallique polyvalent dans lequel p est égal à 2 ou 3 ou 4.

**17.** Composition suivant la revendication 15, **caractérisée en ce que** les dérivés de benzotriazole sont des silanes et/ou des polyorganosiloxanes à fonction benzotriazole comprenant au moins une unité de formule (1) suivante :

$$O_{(3-a)/2}Si\ (R_7)_a-\ G \quad (1)$$

dans laquelle : 1

- $R_7$ représente un radical alkyle en $C_1$-$C_{10}$ éventuellement halogéné ou un radical phényle ou un radical triméthylsilyloxy,

- a est un nombre entier choisi entre 0 et 3 inclusivement,
- et le symbole G désigne un radical monovalent lié directement à un atome de silicium, et qui répond à la formule (2) suivante :

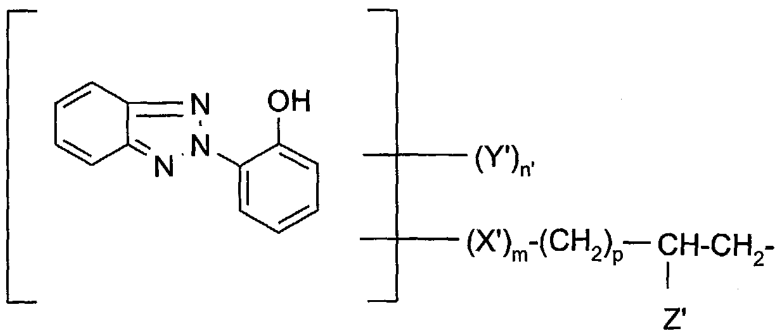

(2)

dans laquelle :

- Y', identiques ou différents, sont choisis parmi les radicaux alkyles en $C_1$-$C_8$, les halogènes et les radicaux alkoxy en $C_1$-$C_4$ étant entendu que, dans ce dernier cas, deux Y' adjacents d'un même noyau aromatique peuvent former ensemble un groupement alkylidène dioxy dans lequel le groupe alkylidène contient de 1 à 2 atomes de carbone,
- X' représente O ou NH,
- Z' représente l'hydrogène ou un radical alkyle en $C_1$-$C_4$,
- n' est un nombre entier compris entre 0 et 3 inclusivement,
- m est 0 ou 1,
- p représente un nombre entier compris entre 1 et 10, inclusivement.

**18.** Composition suivant la revendication précédente, **caractérisée en ce que** le dérivé silicié à fonction benzotriazole répond à l'une des formules (5) ou (6) suivantes :

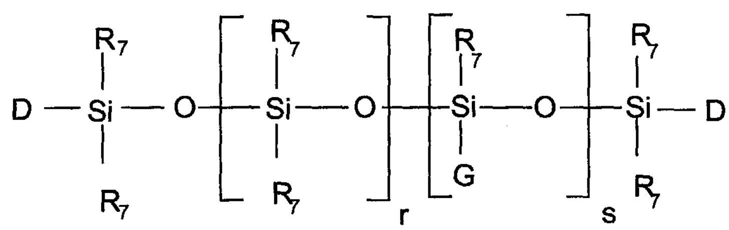

(5)

ou

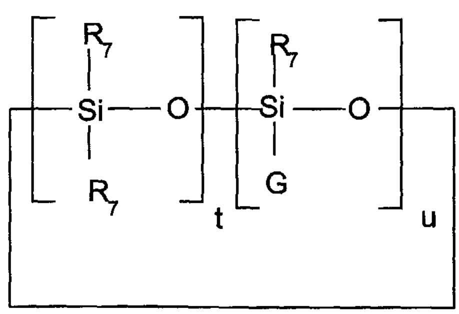

(6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) définie dans la revendication 18.

**19.** Composition suivant la revendication précédente, **caractérisée en ce que** le dérivé silicié à fonction benzotriazole répond à la formule (7) suivante :

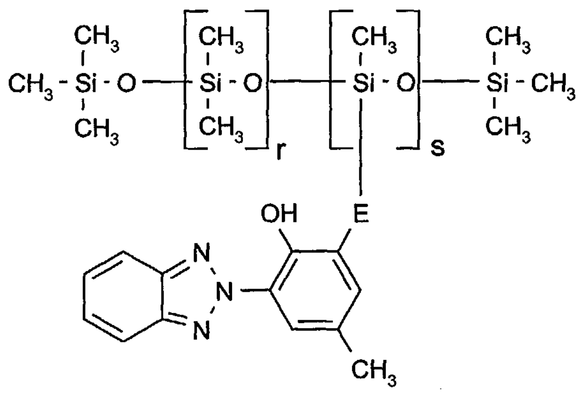

(7)

avec

$$0 \leq r \leq 10,$$

$$1 \leq s \leq 10,$$

et où E reorésente le radical divalent :

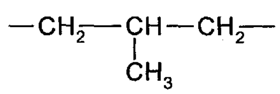

**20.** Composition suivant la revendication précédente, **caractérisée en ce que** le dérivé silicié à fonction benzotriazole est le Drométrizole Trisiloxane de formule suivante :

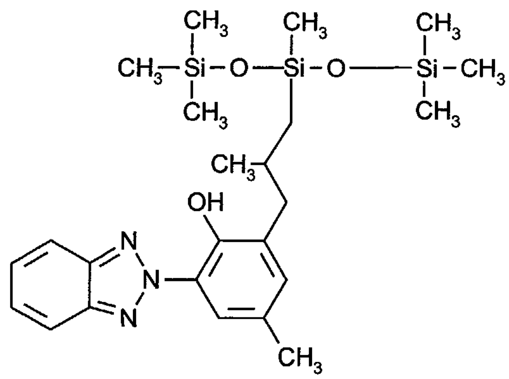

**21.** Composition selon l'une quelconque des revendications 14 à 20, **caractérisée en ce que** les filtres UV sont présents dans la composition dans des proportions allant de 0,1 à 20% en poids par rapport au poids total de la composition.

**22.** Composition selon l'une quelconque des revendications 12 à 21, **caractérisée en ce que** ladite composition renferme en outre au moins un composé choisi parmi les agents hydratants ; les agents dépigmentants ou propigmentants; les agents anti-pollution et/ou anti-radicalaire ; les agents anti-microbiens ; les Inhibiteurs de NO-synthase ; les agents stimulant la synthèse de macromolécules dermiques ou épidermiques et/ou empêchant leur dégradation ; les agents stimulant la prolifération des fibroblastes ou des kératinocytes et/ou la différenciation des kératinocytes ; les agents dermo-décontractants ; les agents tenseurs ; les agents apaisants ; les agents agissant sur la microcirculation et/ou les agents agissant sur le métabolisme énergétique des cellules et leurs mélanges.

**23.** Utilisation d'au moins un dérivé de formule (I) tel que défini dans l'une quelconques des revendications 1 à 10 dans une composition ou pour la fabrication d'une composition, le dit dérivé ou ladite composition étant destiné à induire une photo-protection active de la peau.

**24.** Utilisation suivant la revendication précédente, **caractérisée en ce que** le dit dérivé ou ladite composition est destiné à préparer la peau à recevoir le soleil et/ou à protéger la peau et ses annexes durant ou suite à une exposition aux UV.

**25.** Utilisation d'au moins un dérivé de formule (I) tel que défini dans l'une quelconques des revendications 1 à 10 dans une composition ou pour la fabrication d'une composition, le dit dérivé ou ladite composition étant destiné à favoriser la desquamation de la peau et/ou stimuler le renouvellement épidermique.

**26.** Utilisation suivant l'une quelconque des revendications 24 ou 25, **caractérisée en ce que** le dit dérivé ou ladite composition est destiné à lutter contre le vieillissement cutané intrinsèque et/ou extrinsèque.

**27.** Utilisation cosmétique d'au moins un composé de formule (1) tel que défini dans l'une quelconque des revendications 1 à 10, comme agent stimulant la synthèse intracellulaire de glutathion.

**28.** Procédé de traitement cosmétique de la peau comprenant l'application topique sur ladite peau ou l'administration orale d'une composition selon l'une quelconque des revendications 12 à 22 pour maintenir ou améliorer l'aspect général de la peau d'un individu qui en fait usage.

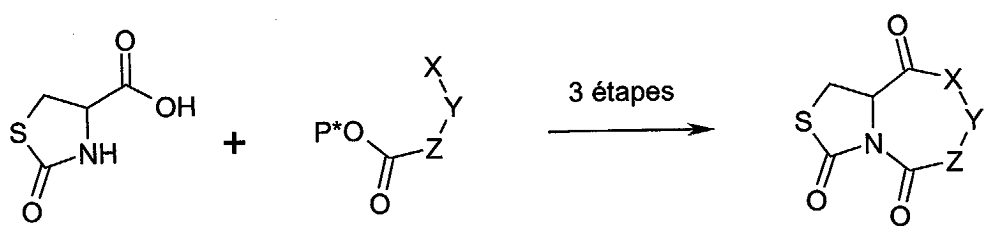


(I)

Fig .1

Office européen des brevets

# RAPPORT DE RECHERCHE EUROPEENNE

Numéro de la demande
EP 04 29 0957

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | EP 0 583 863 A (CLINTEC NUTRITION CO) 23 février 1994 (1994-02-23) * colonne 2, ligne 41 - colonne 2, ligne 51; revendications 1,9,10 * --- | 1-28 | C07D513/04 C07D513/14 A61K31/4985 A61K31/5365 |
| D,A | EP 0 655 245 A (FREE RADICAL SCIENCES INC) 31 mai 1995 (1995-05-31) * page 3, ligne 1 - page 3, ligne 14; revendication 3 * --- | 1-28 | |
| A | EP 0 650 725 A (FREE RADICAL SCIENCES INC) 3 mai 1995 (1995-05-03) * colonne 3, ligne 15 - colonne 3, ligne 33; revendication 1 * --- | 1-28 | |
| A | EP 0 465 191 A (SANTEN PHARMA CO LTD) 8 janvier 1992 (1992-01-08) * revendication 1 * ----- | 1-28 | |

DOMAINES TECHNIQUES RECHERCHES (Int.Cl.7)
C07D
A61K

Le présent rapport a été établi pour toutes les revendications

| Lieu de la recherche | Date d'achèvement de la recherche | Examinateur |
|---|---|---|
| MUNICH | 14 juin 2004 | Wörth, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 04 29 0957

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-06-2004

| Document brevet cité au rapport de recherche | Date de publication | Membre(s) de la famille de brevet(s) | Date de publication |
|---|---|---|---|
| EP 0583863 A | 23-02-1994 | US 5208249 A | 04-05-1993 |
| | | AU 666931 B2 | 29-02-1996 |
| | | AU 3718193 A | 24-02-1994 |
| | | CA 2096036 A1 | 21-02-1994 |
| | | DE 69306063 D1 | 02-01-1997 |
| | | DE 69306063 T2 | 22-05-1997 |
| | | EP 0583863 A1 | 23-02-1994 |
| | | ES 2094479 T3 | 16-01-1997 |
| | | JP 6183973 A | 05-07-1994 |
| EP 0655245 A | 31-05-1995 | AU 7596994 A | 18-05-1995 |
| | | CA 2134707 A1 | 02-05-1995 |
| | | EP 0655245 A2 | 31-05-1995 |
| | | JP 7188018 A | 25-07-1995 |
| EP 0650725 A | 03-05-1995 | AU 7752794 A | 18-05-1995 |
| | | CA 2134950 A1 | 04-05-1995 |
| | | EP 0650725 A1 | 03-05-1995 |
| | | JP 7215868 A | 15-08-1995 |
| EP 0465191 A | 08-01-1992 | DE 69107130 D1 | 16-03-1995 |
| | | DE 69107130 T2 | 03-08-1995 |
| | | EP 0465191 A2 | 08-01-1992 |
| | | JP 2724778 B2 | 09-03-1998 |
| | | JP 5009190 A | 19-01-1993 |
| | | US 5183816 A | 02-02-1993 |
| | | US 5256655 A | 26-10-1993 |

EPO FORM P0460

Pour tout renseignement concernant cette annexe : voir Journal Officiel de l'Office européen des brevets, No.12/82